Europäisches Patentamt

European Patent Office

Office européen des brevets

(19)

(11) Veröffentlichungsnummer: **0 400 617**

**A2**

(12) **EUROPÄISCHE PATENTANMELDUNG**

(21) Anmeldenummer: **90110280.6**

(22) Anmeldetag: **30.05.90**

(51) Int. Cl.5: **A61K 31/18, A61K 31/00**

(30) Priorität: **01.06.89 DE 3917823**

(43) Veröffentlichungstag der Anmeldung:
**05.12.90 Patentblatt 90/49**

(84) Benannte Vertragsstaaten:
**AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Stief, Thomas, Dr.**
**Avda. Kansas City, 28 Bloque, 1.Apt. 225**
**E-41007 Sevilla(ES)**
Erfinder: **Heimburger, Norbert, Prof. Dr.**
**Sonnenhang 10**
**D-3550 Marburg(DE)**
Erfinder: **Reiner, Götz, Dr.**
**Hauptstrasse 33**
**D-6301 Staufenberg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) Oxydantien als systemisches (parenterales) Arzneimittel.

(57) Es wird beschrieben, daß Oxydantien, besonders methionin-selektive Oxydantien als systemische (parenterale) Arzneimittel besonders zur Antikoagulation und Fibrinolyse oder Immunstimulation verwendet werden können.

EP 0 400 617 A2

## Oxydantien als systemisches (parenterales) Arzneimittel

Die Erfindung betrifft Arzneimittel zur systemischen (parenteralen) Anwendung, die Oxydantien, vorzugsweise methioninselektive Oxydantien enthalten, besonders zur Antikoagulation und Fibrinolyse und Immunstimulation.

Substanzen, die antikoagulatorisch wirken und/oder gleichzeitig die Fibrinolyse fördern, sind von hoher klinischer Bedeutung, denn sie eignen sich sowohl für die Prophylaxe von Hyperkoagulabilitäten als auch von hypofibrinolytischen Zuständen.

Zur Antikoagulation werden nach dem Stand der Technik Thrombozytenaggregationshemmer (wie Acetylsalicylsäure), Heparin oder Vitamin K-Antagonisten, zur Fibrinolyse Streptokinase, Urokinase (u-PA) oder Gewebe-Plasminogen-Aktivator (t-PA) eingesetzt. Keine dieser Substanzen besitzt jedoch eine ausgeprägt antikoagulierende und profibrinolytische Eigenschaft.

Es wurde nun überraschenderweise gefunden, daß Oxydantien vom Typ der N-Halogenamine, die über unterchlorige Säure (HOCl) und Wasserstoffsuperoxid ($H_2O_2$) Singlet Oxygen freizusetzen vermögen, der energiereicher als normaler Sauerstoff ist, sowohl stark antikoagulierend als auch profibrinolytisch wirken, wenn diese in Millimol-Konzentrationen Blut zugesetzt werden. Der Terminus Profibrinolyse schließt auch eine Proproteolyse ein, wenn man Collagen und Fibrin als Proteinsubstrate betrachtet. Diese Effekte werden durch Zugabe von Singlet-Oxygen-Föngern wie $NaN_3$ unterdrückt, nicht aber durch Mannitol, das für die Neutralisation von OH-Radikalen verwendet wird.

Oxydantien der Klasse der Chloramine wurden bisher in vitro zur Desinfektion verwendet. In N. Engl. J. Med. 320, 365-376 (1989) ist angegeben, daß Oxydantien nicht intravenös gegeben werden sollten.

Gegenstand der Erfindung ist daher ein Oxydans, besonders ein methioninselektives Oxydans, als systemisch (parenteral) anwendbares Arzneimittel. Diese Arzneimittel können besonders zur Antikoagulation und Fibrinolyse sowie Immunstimulation verwendet werden.

Es ist bekannt, daß Methionin und Cystein in neutralem bis leicht basischem Milieu durch Chloraminderivate selektiv oxidiert werden.

Diese Oxydantien werden in millimolaren Mengen von Kaninchen ohne ernste Nebenwirkungen vertragen (Am.Rev. Resp.Dis. 119 (1979), 953). Chloramine sind als physiologische Substanzen zu betrachten, denn sie werden von aktivierten Makrophagen freigesetzt (Science 222 (1983) 625), um Infektionserreger und Gewebetrümmer anzugreifen und abzuräumen. Darüber hinaus ist bedingt durch die Verwendung der Chloramine zur Wund- und Körperhöhlendesinfektion bekannt, daß diese Oxydantien auch vom Menschen in millimolaren Konzentrationen toleriert werden.

Die Oxydantien können erfindungsgemäß in Dosen verabreicht werden, daß im Körper eine Konzentration von 0,01 bis 0,5 mmol Oxydans pro Liter Blut erreicht wird.

Die Oxydantien, besonders methionin-selektive Oxydantien und ganz besonders Halogenamine sind sowohl zur systemischen und lokalen Antikoagulation als auch zur Fibrinolyse einschließlich lokaler Proteolyse geeignet. Biologisch ist das insofern von Bedeutung, als anoxydiertes Gewebe und auch Bindegewebe durch abbauende Zellen leichter als natives eliminiert werden (Biochemistry 14 (1975), 4497). Dafür sind Chloraminspülungen insbesondere nach chirurgischen Eingriffen indiziert, wenn die Gefahr einer sekundären Briden- und Gewebeverwachsung besteht, die, wie dem Fachmann bekannt ist, sogar zu tödlichen Darmverschlüssen führen kann.

Für die systemische Anwendung von Halogenaminen eignen sich Konzentrationen von 0.01 bis 10, vorzugsweise 0.1 bis 2.5 millimol Halogenamin bezogen auf einen Liter Blut, d.h. die Dosis für einen 75 kg schweren Menschen liegt bei 0.06 bis 60 und vorzugsweise zwischen 0.6 und 15 millimol Halogenamin/Liter Blut.

Für die lokale Applikation von Halogenaminen, z. B. eine Lysebehandlung oder Vermeidung von Verwachsungen, sind entsprechend geringere Dosen indiziert.

Die Erfindung soll an folgenden Beispielen veranschaulicht werden.

Beispiel 1

Antikoagulierender Effekt von Halogenaminen

a) Oxydation

10 ml normales Citratplasma (human) wurde mit folgenden Volumina einer 400 mmol Chloramin T-Lösung versetzt: a) 1000 $\mu$l, b) 500 $\mu$l, c) 250 $\mu$l, d) 125 $\mu$l, e) 62,5 $\mu$l, f) 13 $\mu$l, g) 0 $\mu$l; daraus resultierten Plasmakonzentrationen an Chloramin T von: a) 40 mmol, b) 20 mmol, c) 10 mmol, d) 5 mmol, e) 2,5 mmol, f) 0,5 mmol und g) 0 mmol. Nach zweiminütiger Inkubation bei Raumtemperatur wurde die Oxydation durch Zugabe von jeweils 1 ml 400 mmol N-acetyl-Methionin, pH 8,0 gestoppt und das Plasma getestet.

b) Testung auf Einzelfaktor-Aktivität

In Proben der unterschiedlich stark oxydierten Plasmen (a bis g) wurde mit kommerziell erhältlichen Testkits der Behringwerke die Faktor VIII(F VIII)-, C1-Inhibitor (C1-INH)-, Antithrombin (AT)- und Alpha-2-Antiplasmin (A2-PI)-Aktivität bestimmt. Ergebnis siehe Tabelle 1.

Tabelle 1

| Konzentration Chloramin T im Plasma(mmol) | Aktivitäten | | | |
|---|---|---|---|---|
| | F VIII koagulometrisch | AT | A2-PI | C1-INH |
| | | (% von 0 mmol Chloramin T-Plasma) | | |
| 0 | 100 | 100 | 100 | 100 |
| 0,5 | 91 | 95 | 86 | 100 |
| 2,5 | 60 | 85 | 54 | 95 |
| 5 | 22 | 74 | 31 | 96 |
| 10 | 0 | 65 | 16 | 91 |
| 20 | 0 | 51 | 15 | 94 |
| 40 | 0 | 48 | 15 | 93 |

Man erkennt, daß mit steigenden Chloramin-Konzentrationen im Plasma die Aktivitäten von F VIII und A2-PI stark abfallen; biologisch bedeutet das, daß die Gerinnung gebremst (F VIII ist ein wesentlicher Faktor der Gerinnung) und die Fibrinolyse gefördert wird (A2-PI ist ein Schlüsselinhibitor der Fibrinolyse). Demzufolge wirkt Chloramin antikoagulierend und profibrinolytisch. Hingegen wird die Aktivität von C1-Inhibitor und auch von AT bei diesen Dosierungen nur mäßig beeinträchtigt.

Beispiel 2

Antikoagulatorischer Effekt von Chloramin T auf Plasmafibrinogen

a) in Abhängigkeit von der Inkubationszeit

Lyophilisiertes Standard-Human-Plasma (Behringwerke) wurde in Mischungen aus 800 $\mu$l aqua dest. (37°C) und 200 $\mu$l Chloramin T folgender Konzentrationen gelöst: a) 0, b) 25 mmol, c) 50 mmol (37°C); nach Inkubationszeiten von 30 sec, 1, 2, 3, 5, 7, 10, 13 und 15 min wurden jeweils 100 $\mu$l Plasma mit 100 $\mu$l DBA-Puffer verdünnt, 100 $\mu$l Thrombinreagenz (Behringwerke) zugesetzt und die Thrombinzeit am Schnitger gemessen. Ergebnis siehe Tabelle 2.

Tabelle 2

| Inkubationszeit | Thrombinzeit (Mittelwert aus Doppelbestimmung) | | |
| --- | --- | --- | --- |
| | Chloramin-Konzentration im Plasma | | |
| | 0 mmol | 5 mmol | 10 mmol |
| 30 sec | 21 sec | 41 sec | 106 sec |
| 1 min | 21 sec | 57 sec | 137 sec |
| 2 min | 20 sec | 69 sec | größer 5 min |
| 3 min | 21 sec | 72 sec | größer 5 min |
| 5 min | 22 sec | 83 sec | größer 5 min |
| 7 min | 21 sec | 85 sec | |
| 10 min | 22 sec | 92 sec | |
| 13 min | 22 sec | 97 sec | |
| 15 min | 21 sec | 100 sec | |

b) in Abhängigkeit von der Chloraminkonzentration

Der Versuchsansatz entsprach dem vorausgehenden, allerdings wurde eine Inkubationszeit von konstant 15 min eingehalten und die Plasmaproben auf 0, 2.5, 5, 10, 20 mmol Chloramin T eingestellt. Analog dazu wurde Testfibrinogen der Behringwerke in einer Konzentration, wie es im Plasma vorliegt (0,27 %) in 50 mmol Tris, pH 7,4, gelöst und ebenso behandelt. Dann wurden jeweils 100 $\mu$l Plasma bzw. Fibrinogenlösung mit 100 $\mu$l DBA-Puffer verdünnt, 60 sec bei 37°C inkubiert, 100 $\mu$l Test-Thrombin (3 IU) zugesetzt und die Gerinnungszeit gemessen. Ergebnis siehe Tabelle 3.

Tabelle 3

| Chloramin T | Thrombinzeit | |
| --- | --- | --- |
| | Fibrinogen | Plasma |
| 0 mmol | 41 sec | 19 sec |
| 2,5 mmol | größer 5 min | 26 sec |
| 5 mmol | größer 5 min | 55 sec |
| 10 mmol | größer 5 min | größer 5 min |
| 20 mmol | größer 5 min | größer 5 min |

Deutlich erkennbar führen bereits niedrige Konzentrationen an Chloramin T zu einer stark verlängerten Thrombinzeit sowohl im Plasma als auch in der Fibrinogenlösung. Auch dieses Phänomen kann man als antikoagulierenden Effekt der Chloramine betrachten.

Beispiel 3

Profibrinolytischer und -proteolytischer Effekt von Chloramin T

100 $\mu$l Standard-Human-Plasma (Behringwerke), mit EDTA antikoaguliert, wurden mit 1,25 IE Urokinase in 250 $\mu$l 100 mmol Tris, 100 mmol NaCl, 1 % Polygelin, 0.1 % Triton X 100, pH 8.4 und 200 $\mu$l Chloramin T abgestufter Konzentration (in aqua dest.) versetzt und 45 min bei 37°C inkubiert. Anschließend wurden 500 $\mu$l 0.6 mmol HD-Nva-CHA-Lys-pNA in 480 mmol NaCl zugesetzt und die Substratreaktion bei 405 nm kinetisch verfolgt. Ergebnis siehe Tabelle 4.

Tabelle 4

| Chloramin T-Konzentration (mmol/l) | Plasminaktivität delta A/min |
|---|---|
| 0 | 0.000 |
| 5 | 0.005 |
| 10 | 0.020 |
| 20 | 0.076 |
| 30 | 0.145 |
| 40 | 0.151 |

Mit steigender Konzentration an Chloramin T steigt auch die Plasminaktivität an, die als Maß für die induzierte Fibrinolyse und Proteolyse gewertet werden kann.

Beispiel 4

Verwendung anderer Oxydationsmittel zur Antikoagulation und Profibrinolyse

Beispielhaft wurden auch Chloramin B und $H_2O_2$ in den vorstehenden Beispielen untersucht. Dabei zeigte Chloramin B einen ähnlichen Effekt wie Chloramin T bei gleichen Oxydans-Konzentrationen. $H_2O_2$ war erst in ca. 500fach höheren Konzentrationen so wirksam.

Beispiel 5

Effekt der Oxydation im Plasma auf in vivo Clot-Lyse beim Kaninchen

a) Methodisches Beispiel

Von [R]Nembutal narkotisierten Kaninchen wurde die Vena jugularis sorgfältig freigelegt und anschließend ein 3 cm langer Vicryl[R]-Faden in die Vene eingebracht; anschließend wurde ein 2 cm langes Venen-Segment abgeklemmt, und nachdem dieses sich mit Blut gefüllt hatte, mit einer weiteren Klammer abgedichtet und in dieses Segment Thrombin (0,05 E) injiziert. Nachdem sich der Thrombus über 20 min voll ausgebildet hatte, wurden beide Klammern entfernt. Die Testsubstanz wurde intravenös in eine Randvene des gegenüberliegenden Ohres injiziert. Der Thrombus wurde in bestimmten Intervallen bis zur Reperfusion oder Lyse visuell beobachtet.

In den Experimenten, in denen der Effekt von Chloramin T untersucht wurde, erhielten die Kaninchen dieses durch 35 eine intravenöse Bolus-Injektion. Das Blut für die Bestimmung der Blutzellen und die Erstellung eines TEG sowie der Gerinnungsparameter wurde aus einer zentralen Ohrarterie per Katheder nach 0, 10, 20, 40, 80 und 160 min entnommen.

b) Ergebnisse

Jeweils fünf 2 1/2 kg schwere Kaninchen erhielten:

| Gruppe 1 | 2000 E Urokinase/kg Körpergewicht |
| Gruppe 2 | 25 mg Chloramin T/Tier vor Thrombusbildung |
| Gruppe 3 | 2000 E Urokinase/kg Körpergewicht + 25 mg Chloramin T/Tier |
| Gruppe 4 | 25 mg Chloramin T/Tier nach Thrombusbildung |

Die Lysezeit für die Tiere der Gruppe 1 betrug im Mittel 91,2 ± 25 min. Bei den Tieren der Gruppe 2 bildete sich überraschenderweise überhaupt kein Thrombus aus, wenn das Chloramin vor der Induktion des Thrombus appliziert wurde. Daher wurde in der Gruppe 3 Chloramin T 15 min nach Thrombusinduktion injiziert und 5 min später die Urokinase. Die Lysezeit für die Kombination beider Präparate lag bei 34,6 ± 9,2 min. Im Vergleich dazu wurde für die Tiere der Gruppe 4, die nur Chloramin T nach Thrombusbildung bekamen, eine Reperfusionszeit entsprechend 33 ± 16 min gemessen.

Die Ergebnisse zeigen, daß Chloramin T allein eine stark thrombolytische Aktivität besitzt, die durch Urokinase nicht verstärkt werden kann. Besondere Bedeutung kommt der Tatsache zu, daß diese Wirkung in vivo ohne Beeinträchtigung der bekannten globalen Gerinnungsparameter und des TEG erzielt werden konnte. Lediglich ein milder und vorübergehender Abfall an Leukozyten und Plättchen wurde beobachtet, der sich über einen Zeitraum von 10 -40 min erstreckte und dann wieder verschwand. Dieser für den fibrinolytischen Effekt charakteristische Leukozytenabfall war auch zu beobachten bei Applikation von 125 mg, 2.5 mg und 0.25 mg Chloramin-T i.v.

## Ansprüche

1. Ein Oxydans als systemisch anwendbares Arzneimittel.

2. Arzneimittel nach Anspruch 1, dadurch gekennzeichnet, daß das Oxydans ein methionin-selektives Oxydans ist.

3. Arzneimittel nach Anspruch 1, dadurch gekennzeichnet, daß das Oxydans ein Halogenamin ist.

4. Arzneimittel nach Anspruch 1, dadurch gekennzeichnet, daß es 0.05-100 mmol des Oxydans pro Dosis (75 kg schwerer Patient) enthält.

5. Verwendung eines Oxydans zur Herstellung eines systemisch anwendbaren Arzneimittels.

6. Verwendung nach Anspruch 5, dadurch gekennzeichnet, daß ein Arzneimittel zur Antikoagulation und Fibrinolyse oder Immunstimulation hergestellt wird.

7. Verwendung nach Anspruch 5, dadurch gekennzeichnet, daß das Oxydans ein reaktives Halogenamin ist.

8. Verwendung nach Anspruch 5, dadurch gekennzeichnet, daß das Oxydans ein Chloramin ist.

9. Verwendung nach Anspruch 5, dadurch gekennzeichnet, daß ein Arzneimittel zur lokalen und systemischen Fibrinolyse hergestellt wird.

10. Verwendung nach Anspruch 5, dadurch gekennzeichnet, daß ein Arzneimittel zur lokalen Förderung von Proteolyse hergestellt wird.

11. Verwendung nach Anspruch 5, dadurch gekennzeichnet, daß 0.05-100 mol Oxydans/Dosis (75 kg schwerer Patient) verwendet wird.

Patentansprüche für folgende Vertragsstaaten: ES, GR

1. Verfahren zur Herstellung eines systemisch anwendbaren Arzneimittels enthaltend ein Oxydans, dadurch gekennzeichnet, daß ein Oxydans in eine für eine systemische Anwendung geeignete Form gebracht wird.

2. Verfahren nach Anspruch 1, dadurch gekennzeichnet, daß das Oxydans ein methionin-selektives Oxydans ist.

3. Verfahren nach Anspruch 1, dadurch gekennzeichnet, daß ein systemisch anwendbares Arzneimittel zur Antikoagulation und Fibrinolyse oder Immunstimulation hergestellt wird.

4. Verfahren nach Anspruch 1, dadurch gekennzeichnet, daß das Oxydans ein reaktives Halogenamin ist.

5. Verfahren nach Anspruch 1, dadurch gekennzeichnet, daß das Oxydans ein Chloramin ist.

6. Verfahren nach Anspruch 1, dadurch gekennzeichnet, daß ein Arzneimittel zur lokalen und systemischen Fibrinolyse hergestellt wird.

7. Verfahren nach Anspruch 1, dadurch gekennzeichnet, daß ein Arzneimittel zur lokalen Förderung von Proteolyse hergestellt wird.

8. Verfahren nach Anspruch 1, dadurch gekennzeichnet, daß 0.2-100 mmol Oxydans/Dosis (75 kg schwerer Patient) verwendet wird.